# EUROPÄISCHE PATENTSCHRIFT

(11) **EP 0 570 559 B1**
(45) Veröffentlichungstag und Bekanntmachung des Hinweises auf die Patenterteilung: **10.04.1996**
(21) Anmeldenummer: 92924677.5
(22) Anmeldetag: 05.12.1992
(51) Int. Cl.: A61K 6/00, C09J 4/06

(54) **HAFTFESTE, WASSERUNDURCHLÄSSIGE UND HYDROLYSEBESTÄNDIGE VERBUNDSCHICHT FÜR METALL-, KERAMIK-, GLAS-, POLYMER-KUNSTSTOFF-VERBUNDE UND DISPERSION ZU DEREN HERSTELLUNG**
HIGHLY BONDING, WATER-IMPERMEABLE AND HYDROLYSIS-RESISTANT BINDING LAYER FOR METAL, CERAMIC, GLASS, POLYMER-PLASTIC COMPOSITE MATERIALS, AND DISPERSION FOR PRODUCING THE SAME
COUCHE DE LIAISON RESISTANTE A L'HYDROLYSE, IMPERMEABLE A L'EAU ET ASSURANT UNE FIXATION SOLIDE POUR MATERIAUX COMPOSITES PLASTIQUE-METAL, PLASTIQUE-CERAMIQUE, PLASTIQUE-VERRE, PLASTIQUE-POLYMERE, ET DISPERSION UTILE POUR PREPARER CELLE-CI

(30) Priorität: 09.12.1991 DE 4140504; 27.08.1992 DE 4228530
(43) Veröffentlichungstag der Anmeldung: 24.11.1993
(73) Patentinhaber: HERAEUS KULZER GMBH, D-63450 Hanau (DE)
(72) Erfinder: GÖBEL, Roland, D-6900 Jena (DE)
(74) Vertreter: Kühn, Hans-Christian
(86) Internationale Anmeldenummer: EP9202820
(87) Internationale Veröffentlichungsnummer: WO9311732

(56) Entgegenhaltungen:
- EP-A- 0 223 067
- FR-A- 2 227 309

## Beschreibung

Die Erfindung betrifft eine Dispersion zur Herstellung einer haftfesten, wasserundurchlässigen und hydrolysebeständigen Verbundschicht für Metall-, Keramik-, Glas-, Polymer-Kunststoff-Verbunde. Vorzugsweise findet die erfindungsgemäße Dispersion Verwendung zur Herstellung von Verbundschichten, die ständigen mechanischen und durch Temperaturwechsel bedingten Beanspruchungen unter gleichzeitiger Feuchteeinwirkung unterliegen, hier besonders Verbundschichten für die Dentaltechnik.

In den letzten Jahren sind zahlreiche Vorschläge gemacht worden, Kunststoffe spaltfrei mit Metalloberflächen zu verbinden. Grundprinzip dieser Verfahren ist, daß in einem ersten Schritt eine anorganische (meist silikatische) Schicht auf eine Metalloberfläche aufgebracht (Silikatisierung) und in einem zweiten Schritt die Oberfläche mit einem funktionellen Alkoxysilan (Silanisierung) bestrichen wird. Dabei stellt dieses Haftsilan (meist hydrolysiertes γ-Methacryloyloxypropyltrimethoxysilan) das Bindeglied zwischen der anorganischen silikatischen Schicht und einem methacrylathaltigen Dentalkunststoff dar, wobei einerseits die freien OH-Gruppen des Haftsilans mit den Oberflächen-OH-Gruppen der Silikatschicht unter Kondensationsreaktionen an die Silikatschicht chemisch gebunden werden, während andererseits über die Methacrylatgruppe des Haftsilans eine Anbindung z.B. an einen Dentalkunststoff erfolgt. Die bekannten Verfahren unterscheiden sich durch die Methoden des unterschiedlichen Aufbringens der Silikatschicht, während das Aufbringen des Haftsilans bei allen Verfahren nahezu identisch ist.

US-A-4 364 731 beschreibt ein Verfahren zum Aufbringen einer Siliziumdioxidschicht auf metallische Dentalprothesenteile durch eine Hochfrequenz-Magnetron-Sputtervorrichtung. Da hierfür eine Vakuumbeschichtungsanlage benötigt wird, ist das Verfahren mit einem erheblichen apparativen Aufwand verbunden.

In DE-C- 34 03 894 erfolgt das Aufbringen der Silikatschicht durch einen deutlich einfacheren Flammenhydrolyseprozeß des Tetraethoxysilans, wobei allerdings gute Haftfestigkeiten des Kunststoffs auf dem Metall nur bei genauer Einhaltung der Geräteparameter erhalten werden.

Weiterhin wird in DD 276 453 ein Verfahren beschrieben, bei dem eine Silikat-Chromoxid-Schicht durch eine Sol-Gel-Lösung auf eine Dentallegierungsoberfläche aufgebracht und durch einen nachfolgenden Temperprozeß (320°C, 2 - 8 min) verfestigt wird. Als kritisch erweisen sich dabei vor allem Dentallegierungen mit einem höheren Kupfergehalt (>5 Masse-%), da dann durch den Temperprozeß an der Legierungsoberfläche schlecht haftendes Kupfer(II)-oxid gebildet wird, wodurch der Metall-Kunststoff-Verbund geschwächt wird.

In DE-C-38 02 043 wird ein Verfahren beschrieben, bei dem die Silikatschicht durch einen Sandstrahlprozeß aufgebracht wird, indem dem Strahlkorund eine gewisse Menge Siliziumdioxid einer mittleren Partikelgröße ≦5 µm zugesetzt wird. Im Aufschlagbereich der Korundteilchen treten dabei örtlich Energiedichten auf, die ausreichend sind, die feinteiligen Silikatpartikel auf die metallische Oberfläche aufzuschmelzen. Auch mit diesem Verfahren sind ausreichende Verbundfestigkeiten nur durch genaueste Einhaltung der Bedienungsparameter zu erreichen.

Die beschriebenen bekannten Verfahren erfordern alle einen teuren apparativen Geräte- bzw. Chemikalienaufwand und mehrere Prozeßschritte. Außerdem sind die Si-O-Si-Bindungen der Silikatschicht bei Verwendung im Dentalbereich durch die Feuchtebelastung der Kunststoff-Metall-Verbunde im Mundmilieu einem ständigen Hydrolyseangriff ausgesetzt, was bei längerer Einwirkung zu einer Schwächung der Verbundfestigkeit führen kann.

Der Erfindung liegt somit die Aufgabe zugrunde, ein Mittel zu finden, aus dem sich mit nur geringem apparativem Aufwand und preiswert haftfeste, wasserundurchlässige und hydrolysebeständige Verbundschichten herstellen lassen. Die Verbundschichten sollen geeignet sein, Kunststoffe, insbesondere methacrylathaltige Dentalkunststoffe bzw. Klebstoffe, dauerhaft und mit hoher Haftfestigkeit randspaltfrei an Metall-, Glas-, Keramik- bzw. organische Oberflächen zu binden.

Die Aufgabe der Erfindung wird dadurch gelöst, daß einer Phenolharz-Dispersion, die zumindest Phenol mit freien Methylolgruppen, dispergiertes Acrylat, destilliertes Wasser und Aceton enthält, eine oder mehrere ein- bzw. mehrfunktionelle Methacrylatverbindungen zugesetzt sind.

Es wird eine Dispersion eingesetzt, die erfindungsgemäß aus einer Phenolharz-Dispersion (neben Phenol als Ausgangsmonomer für die Formaldehydreaktion können selbstverständlich auch Phenolderivate, wie Kresole und Resorcin, als Reaktionspartner verwendet werden) mit hydroxymethyliertem Phenol mit freien Methylolgruppen, Polyvinylformal bzw. Polyvinylbutyral als plastifizierende Komponente, dispergiertem Acrylat, destilliertem Wasser und Aceton besteht. Dieser Dispersion werden eine oder mehrere ein- bzw. mehrfunktionelle Methacrylatverbindungen zugesetzt. Bei diesen polymerisationsfähigen olefinisch ungesättigten Monomeren kann es sich um Urethanmethacrylate und/oder Hydroxymethacrylate bzw. andere ein- und mehrfunktionelle Methacrylate handeln.

Die Dispersion kann zusätzlich noch 1 bis 10 g eines Alkoxysilans enthalten. Dabei handelt es sich um ein Alkoxysilan mit drei, zwei oder einer Methoxy- bzw. Ethoxygruppe(n) und mindestens einer funktionellen organischen Gruppe, besonders einer Vinyl-, Methacrylat- oder Epoxygruppe. Bevorzugt werden Vinyltrimethoxysilan, Methacrylsäure-3-trimethoxysilylpropylester (γ-Methacryloyloxypropyltrimethoxysilan), 3-Glycidoxypropyltriethoxysilan und Aminoethylaminopropyltrimethoxysilan.

Insbesondere enthält eine für die Erfindung zu verwendende Phenolharz-Dispersion, bezogen auf 100 ml Dispersion, 0,5 bis 5 g Phenolharz und Phenol mit freien Methylolgruppen, 0,05 bis 0,5 g Polyvinylformal bzw. Polyvinylbutyral, 1 bis 5 g dispergiertes Acrylat, 10 bis 30 ml destilliertes Wasser und 10 bis 30 ml Aceton, wobei der Gehalt an Phenol mit freien Methylolgruppen, bezogen auf die Dispersion, 0,05 bis 0,2 Masse-% beträgt. Dieser Dispersion werden 2 bis 20 g einer oder mehrerer ein- bzw. mehrfunktioneller Methacrylatverbindungen zugesetzt, so daß im Ergebnis eine erfindungsgemäße Phenolharz-Methacrylat-Dispersion entsteht.

Diese Dispersion wird auf eine Metall-, Glas-, Keramik- bzw. organische Oberfläche aufgestrichen und einer Temperaturbehandlung zwischen 120 bis 220°C unterzogen. Dabei kondensieren die Methylolphenole über die Resol- und Resitolharze schließlich zu raumvernetzten Resitharzen. Die Methacrylatmonomere können durch basekatalytische Additionsreaktionen ihrer Methylengruppe an die Carbonylfunktion des Aldehyds (Aldoladdition) bzw. durch interpenetrierende Netzwerke in dieses Phenolharzraumnetz eingebunden werden. Es bilden sich haftfeste, wasserundurchlässige und hydrolysebeständige Verbundschichten.

Gegenüber dem Stand der Technik weisen die erfindungsgemäße Dispersion und die damit erzeugte Verbundschicht folgende Vorteile auf:
- Die Verbundschicht verhindert den Verbund schwächende Wasserdiffusion über einen Kunststoff bzw. Klebstoff zu einer z.B. Metallegierungsoberfläche.
- Das Verfestigen der Dispersion erfolgt bei relativ niedriger Temperatur (120-220°C), so daß es z.B. an kritischen Dentallegierungen zu keinen Verfärbungen oder Verzunderungen kommt.
- Die Dispersion ist völlig unabhängig von den in Kontakt zu bringenden Verbundmaterialien.
- Es wird keine zusätzliche Haftvermittlerlösung benötigt, da die Haftvermittlermoleküle bereits in der Verbundschicht integriert sind.
- Die Verbundschicht läßt sich ohne großen apparativen Aufwand, z. B. mit einfachen Wärmequellen, problemlos in jedem Anwendungsbereich ohne aufwendige Einhaltung von Prozeßparametern realisieren.

Zum Stand der Technik wird noch auf FR-A-2 227 309 verwiesen. Hieraus ist ein durch Kontaktdruck zu verklebender Kontaktklebstoff bekannt, der aus einer Mischung eines Acrylat-Latex mit einer wässerigen Phenolharz-Dispersion besteht.

In den folgenden Ausführungsbeispielen wird die Erfindung näher erläutert. Die im weiteren aufgeführten Zug- bzw. Druckscherfestigkeitswerte von Metall-Kunststoff-Verbunden bzw. Metall-Metall-Verklebungen dienen der Bestätigung der Erfindung, ohne jedoch die Art des eintretenden Haftmechanismus näher erklären zu können, da hier bisherige Erklärungen zur Haftung revidiert werden müssen.

Als ein Vergleichswert der Verbundfestigkeit dient die unkonditionierte Oberfläche (Leerwert), wobei diese Oberfläche, wie auch die zum Einsatz der Erfindung dienenden Oberflächen, zunächst gesandstrahlt wird (Aluminiumkorund 50 bis 250 µm, 1 - 5 bar). Ein weiterer Vergleichswert ist die Haftfestigkeit, die erreicht wird, wenn die Festkörperoberflächen lediglich mit einem Teil der erfindungsgemäßen Dispersion (Dispersion A) beschichtet werden. Ein dritter Vergleichswert sind die Scherfestigkeiten, die mit der erfindungsgemäßen Dispersion erzielt werden.

Zusammensetzung der Dispersion A:
- 0,75 g Phenolharz und Phenol mit freien Methylolgruppen
- 0,1 g Polyvinylformal bzw. Polyvinylbutyral
- 2 g dispergiertes Acrylat
- 20 ml destilliertes Wasser
- 60 ml Isopropanol
- 20 ml Aceton.

Zu 100 ml dieser Dispersion, deren Gehalt an Phenol mit freien Methylolgruppen 0,1 Masse-% beträgt, werden zur Erzielung der erfindungsgemäßen Zusammensetzung - Dispersion B -
- 2 g Methacrylsäure-3-trimethoxysilylpropylester
- 5 g Methylmethacrylat
- 2 g Triethylenglykoldimethacrylat
- 1 g 1,4-Butandioldimethacrylat
- 1 g Trimethylolpropantriacrylat
- 0,1 g Campherchinon
- 0,2 g Triethanolamin
zugegeben.

Die Dispersion B und zur Schaffung der genannten Vergleichswerte auch die Dispersion A allein wird im Beispiel auf gesandstrahlte Dentallegierungen aufgebracht und einer ca. 15-minütigen Temperaturbehandlung bei 120 bis 220°C, vorzugsweise 150°C, unterzogen. Im weiteren kann auf diese Oberfläche ein lichthärtender Opaker auf Methacrylat-Basis aufgetragen und polymerisiert werden. Daran anschließend wird aus lichthärtendem Kunststoff ein Zylinder (im Beispiel mit 5 mm Durchmesser und 2 mm Höhe) aufmodelliert und ebenfalls polymerisiert. In diesem Fall ist es von Vorteil, der erfindungsgemäßen Dispersion 0,1 bis 1 Masse-% von photoaktiven Komponenten, wie das System aus Campherchinon und Triethanolamin bzw. N,N-Dimethylaminoethylmethacrylat o.ä., zuzusetzen, was eine weitere Haftverbesserung bewirkt. Diese so hergestellten Metall-Kunststoff-Verbunde werden eine Stunde in destilliertem Wasser gekocht, 24 Stunden in Wasser gelagert und danach in einem Druck-Scher-Versuch (Vorschubgeschwindigkeit 1 mm min⁻¹) auf Verbundfestigkeit getestet. Die erzielten Verbundfestigkeitswerte sind in Tabelle 1 und 2 aufgeführt.

**Tabelle 1**

| Metall-Kunststoff-Verbund (Kunststoff: Dentacolor) | | | |
|---|---|---|---|
| Dentallegierung | Scherfestigkeit Leerwert | Scherfestigkeit Dispersion A | Scherfestigkeit Dispersion B |
| Maingold | 11 MPa | 12 MPa | 16 MPa |
| Degulor | 10,5 MPa | 11 MPa | 15,5 MPa |
| Palliag | 9 MPa | 11 MPa | 18 MPa |
| Auropal 2 | 10,8 MPa | 11,2 MPa | 16,5 MPa |
| Duo Pal 6 | 8,8 MPa | 10,4 MPa | 16,5 MPa |
| Simidur | 7,9 MPa | 10,2 MPa | 16,9 MPa |
| Wiron 88 | 7,2 MPa | 9,5 MPa | 15,3 MPa |

**Tabelle 2**

| Metall-Kunststoff-Verbund (Metall: Wiron 88) | | | |
|---|---|---|---|
| Dentalkunststoff | Scherfestigkeit Leerwert | Scherfestigkeit Dispersion A | Scherfestigkeit Dispersion B |
| P 50 | 15,4 MPa | 16,7 MPa | 28,5 MPa |
| Estilux | 15,1 MPa | 16,8 MPa | 26,8 MPa |
| Visio-Molar | 13,4 MPa | 15,2 MPa | 25,6 MPa |
| Charisma | 12,1 MPa | 14,8 MPa | 23,2 MPa |
| Heliomolar | 11,2 MPa | 12,5 MPa | 19,5 MPa |

Neben den in den Tabellen 1 und 2 angegebenen Scherfestigkeitswerten für Metall-Kunststoff-Verbunde dienen die in Tabelle 3 aufgeführten Zug-Scherfestigkeitswerte von Metall-Metall-Verklebungen der weiteren Bestätigung des erfindungsgemäßen Erfolges. Die Konditionierung der Metalloberflächen erfolgt dabei analog der bereits zur Erstellung der Metall-Kunststoff-Verbunde beschriebenen Vorgehensweise. Die Überlappungslänge der Klebung beträgt dabei 10 mm und die Klebefläche 50 mm². Diese Verklebungen wurden 10 Stunden gekocht.

**Tabelle 3**

| Metall-Metall-Verklebung (Metall: Wiron 88) | | | |
|---|---|---|---|
| Klebstoff | Scherfestigkeit Leerwert | Scherfestigkeit Dispersion A | Scherfestigkeit Dispersion B |
| Chemiace | 12,8 MPa | 14,5 MPa | 28,5 MPa |
| Brilliant enamel kit | 14,4 MPa | 16,3 MPa | 25,2 MPa |
| Palavit 55 | 12,1 MPa | 13,8 MPa | 25,4 MPa |
| Microfill pontic | 11,2 MPa | 13,2 MPa | 20,8 MPa |
| Helapox blau | 9,5 MPa | 20,2 MPa | 19,5 MPa |

Im Vergleich zu den methacrylathaltigen Dentalklebstoffen Chemiace, Brilliant enamel kit, Palavit 55 und Microfill pontic nimmt Helapox blau (Epoxidharz) eine gewisse Sonderstellung ein.

Sowohl die Zug-Scherfestigkeiten der Metall-Metall-Verklebungen als auch die Druck-Scherfestigkeiten der Metall-Kunststoff-Verbunde zeigen, daß mit der erfindungsgemäßen Dispersion eine um etwa 100% höhere Haftfestigkeit gegenüber den unkonditionierten Oberflächen zu erreichen ist. Der Bruch stellt sich als reiner Kohäsionsbruch dar.

Durch die Erfindung steht eine haftfeste, wasserundurchlässige und hydrolysebeständige Verbundschicht zur Verfügung, die sich für die vielfältigsten Verbunde eignet und nicht auf die Verwendung in der Dentaltechnik beschränkt ist. Eine ebenso vorteilhafte Verwendung der erfindungsgemäßen Dispersion ist im Maschinen- und Karosseriebau gegeben. Die Dispersion wird dort zur Herstellung einer Lackunterschicht angewandt, wodurch eine deutliche Verlängerung der Lebensdauer der Lack-Verbundschichten selbst unter tropischen Bedingungen erreichbar ist. Für diesen Zweck hat es sich bewährt, der Dispersion Lack-Bestandteile zuzugeben; zur Verfestigung wird die Lackunterschicht einer Temperaturbehandlung unterworfen.

## Patentansprüche

1. Dispersion zur Herstellung einer haftfesten, wasserundurchlässigen und hydrolysebständigen Verbundschicht für Metall-, Keramik-, Glas-, Polymer-Kunststoff-Verbunde, dadurch gekennzeichnet, daß einer Phenolharz-Dispersion, die zumindest Phenol mit freien Methylolgruppen, dispergiertes Acrylat, destilliertes Wasser und Aceton enthält, eine oder mehrere ein- bzw. mehrfunktionelle Methacrylatverbindungen zugesetzt sind.

2. Dispersion nach Anspruch 1, dadurch gekennzeichnet, daß 100 ml einer Phenolharz-Dispersion, die 0,5 bis 5 g Phenolharz und Phenol mit freien Methylolgruppen, 0,05 bis 0,5 g Polyvinylformal bzw. Polyvinylbutyral, 1 bis 5 g dispergiertes Acrylat, 10 bis 30 ml destilliertes Wasser und 10 bis 30 ml Aceton enthält, 2 bis 20 g einer oder mehrerer ein- bzw. mehrfunktioneller Methacrylatverbindungen zugesetzt sind.

3. Dispersion nach Anspruch 1 oder 2, dadurch gekennzeichnet, daß es sich bei den Methacrylatverbindungen um Urethanmethacrylate und/oder Hydroxymethacrylate bzw. andere ein- bzw. mehrfunktionelle Methacrylate handelt.

4. Dispersion nach einem der Ansprüche 1 bis 3, dadurch gekennzeichnet, daß der Dispersion 1 bis 10 g eines Alkoxysilans zugesetzt sind.

5. Dispersion nach Anspruch 4, dadurch gekennzeichnet, daß es sich bei dem Alkoxysilan um ein solches mit drei, zwei oder einer Methoxy- bzw. Ethoxygruppe und mindestens einer funktionellen organischen Gruppe handelt.

6. Dispersion nach Anspruch 5, dadurch gekennzeichnet, daß es sich bei der funktionellen organischen Gruppe des Alkoxysilans um eine Vinyl-, Methacrylat- oder Epoxygruppe handelt.

7. Dispersion nach einem der Ansprüche 4 bis 6, dadurch gekennzeichnet, daß es sich bei dem Alkoxysilan um Vinyltrimethoxysilan, Methacrylsäure-3- trimethoxysilylpropylester, 3-Glycidoxypropyltriethoxysilan oder Aminoethylaminopropyltrimethoxysilan handelt.

8. Dispersion nach einem der Ansprüche 1 bis 7, dadurch gekennzeichnet, daß der Dispersion 0,1 bis 1 Masse-% einer photoaktiven Komponente, bezogen auf die Masse der Methacrylatverbindungen, zugesetzt sind.

9. Dispersion nach Anspruch 8, dadurch gekennzeichnet, daß die photoaktive Komponente ein photoaktives System aus Campherchinon und Triethanolamin bzw. N,N-Dimethylaminoethylmethacrylat ist.

10. Dispersion nach einem der Ansprüche 1 bis 9, dadurch gekennzeichnet, daß die Dispersion einen pH-Wert zwischen 7,2 und 8 aufweist.

11. Verfahren zur Herstellung einer haftfesten, wasserundurchlässigen und hydrolysebeständigen Verbundschicht für Metall-, Keramik-, Glas-, Polymer-Kunststoff-Verbunde unter Anwendung der methacrylathaltigen Phenolharz-Dispersion nach einem der Ansprüche 1 bis 10, dadurch gekennzeichnet, daß die Phenolharz-Dispersion auf einem Träger aufgebracht und einer Temperaturbehandlung im Bereich von 120 bis 220° C unterworfen wird und daß danach auf die so erhaltene Schicht ein weiterer Stoff des Verbundes aufgebracht wird.

12. Verfahren nach Anspruch 11, dadurch gekennzeichnet, daß die erhaltene Schicht mit einem Opaker auf Methacrylat-Basis versehen wird.

## Claims

1. A dispersion for the production of a strongly adhesive water-impermeable and hydrolysis resistant bonding layer for metal, ceramics, glass, polymer -plastics composites, characterised in that one or more mono- or polyfunctional methacrylate compounds are added to a phenolic resin dispersion, which contains at least phenol with free methylol groups dispersed acrylate, distilled water and acetone.

2. A dispersion according to Claim 1, characterised in that 2 to 20 g of one or more mono- or polyfunctional methacrylate compounds are added to 100 ml of a phenolic resin dispersion, which contains 0.5 to 5 g phenolic resin and phenol with free methylol groups, 0.05 to 0.5 g polyvinylformal or polyvinylbutyral, 1 to 5 g dispersed acrylate, 10 to 30 ml distilled water and 10 to 30 ml acetone.

3. A dispersion according to Claim 1 or 2, characterised in that the methacrylate compounds are urethane methacrylates and/or hydroxymethacrylates or other mono- or polyfunctional methacrylates.

4. A dispersion according to one of Claims 1 to 3, characterised in that 1 to 10 g of an alkoxysilane are added to the dispersion.

5. A dispersion according to Claim 4, characterised in that the alkoxysilane is one having three, two or one methoxy- or ethoxy groups and at least one functional organic group.

6. A dispersion according to Claim 5, characterised in that the functional organic group of alkoxysilane is a vinyl-, methacrylate- or epoxy group.

7. A dispersion according to one of Claims 4 to 6, characterised in that the alkoxysilane is vinyltrimethoxysilane, methacrylic acid-3-trimethoxysilylpropylester, 3-glycidoxypropyltriethoxysilane or aminoethylaminopropyltrimethoxysilane.

8. A dispersion according to one of Claims 1 to 7, characterised in that 0.1 to 1 % by mass of a photoactive component, based on the mass of the methacrylate compounds, is added to the dispersion.

9. A dispersion according to Claim 8, characterised in that the photoactive component is a photoactive system of camphor quinone and triethanolamine or N,N-dimethylaminoethylmethacrylate.

10. A dispersion according to one of Claims 1 to 9, characterised in that the dispersion has a pH value of between 7.2 and 8.

11. A method for the production of a strongly adhesive, water-impermeable and hydrolysis resistant bonding layer for metal, ceramic, glass, polymer -plastic composites with the use of the phenolic resin dispersion containing methacrylate according to one of Claims 1 to 10, characterised in that the phenolic resin dispersion is applied onto a carrier and is subjected to a temperature treatment in the range of 120 to 220° C and that thereafter a further material of the composite is applied onto the layer which is thus obtained.

12. A method according to Claim 11, characterised in that the layer which is obtained is provided with an opaquer based on methacrylate.

## Revendications

1. Dispersion pour la production d'une couche de liaison présentant une solidité de fixation, étanche à l'eau et résistant à une hydrolyse, destinée aux composites métal-matière synthétique, céramique-matière synthétique, verre-matière synthétique et polymère-matière synthétique, caractérisée en ce qu'on ajoute à une dispersion de résine phénolique, qui contient au moins du phénol présentant des groupements méthylol libres, de l'acrylate dispersé, de l'eau distillée et de l'acétone, un ou plusieurs composés méthacrylate monofonctionnels ou polyfonctionnels.

2. Dispersion selon la revendication 1, caractérisée en ce qu'on ajoute à 100 ml d'une dispersion de résine phénolique, qui contient de 0,5 à 5 g de résine phénolique et de phénol présentant des groupements méthylol libres, de 0,05 à 0,5 g de polyvinylformal ou polyvinylbutyral, de 1 à 5 g d'acrylate dispersé, de 10 à 30 ml d'eau distillée et 10 à 30 ml d'acétone, 2 à 20 g d'un ou plusieurs composés méthacrylate monofonctionnels ou polyfonctionnels.

3. Dispersion selon la revendication 1 ou 2, caractérisée en ce qu'il s'agit, dans le cas des composés méthacrylate, de méthacrylates d'uréthane et/ou d'hydroxyméthacrylates ou d'autres méthacrylates monofonctionnels ou polyfonctionnels.

4. Dispersion selon l'une des revendications 1 à 3, caractérisée en ce qu'on ajoute à la dispersion de 1 à 10 g d'un alcoxysilane.

5. Dispersion selon la revendication 4, caractérisée en ce qu'il s'agit, dans le cas de l'alcoxysilane, d'un alcoxysilane présentant trois, deux ou un groupement(s) méthoxy ou éthoxy et au moins un groupement organique fonctionnel.

6. Dispersion selon la revendication 5, caractérisée en ce qu'il s'agit, dans le cas du groupement organique fonctionnel de l'alcoxysilane, d'un groupement vinyle, méthacrylate ou époxy.

7. Dispersion selon l'une des revendications 4 à 6, caractérisée en ce qu'il s'agit, dans le cas de l'alcoxysilane, du vinyltriméthoxy-silane, du méthacrylate de 3-triméthoxysilylpropyle, du 3-glycidoxypropyltriéthoxysilane ou de l'aminoéthylaminopropyltriméthoxysilane.

8. Dispersion selon l'une des revendications 1 à 7, caractérisée en ce qu'on ajoute à la dispersion de 0,1 à 1% en masse d'un composant photoactif, par rapport à la masse des composés méthacrylate.

9. Dispersion selon la revendication 8, caractérisée en ce que le composant photoactif est un système photoactif constitué de camphre-quinone et de triéthanolamine ou méthacrylate de N,N-diméthylaminoéthyle.

10. Dispersion selon l'une des revendications 1 à 9, caractérisée en ce que la dispersion présente un pH compris entre 7,2 et 8.

11. Procédé de production d'une couche de liaison présentant une solidité de fixation, étanche à l'eau et résistant à une hydrolyse, destinée aux composites métal-matière synthétique, céramique-matière synthétique, verre-matière synthétique et polymère-matière synthétique, en appliquant la dispersion de résine phénolique contenant du méthacrylate selon l'une que des revendications 1 à 10, caractérisé en ce que la dispersion de résine phénolique est disposée sur un support et est soumise à un traitement thermique dans la gamme de 120 à 220°C et en ce qu'une autre matière du composite est disposée sur la couche ainsi obtenue.

12. Procédé selon la revendication 11, caractérisé en ce que la couche obtenue est dotée d'un opacifiant à base de méthacrylate.
